(19) Europäisches Patentamt / European Patent Office / Office européen des brevets

(11) EP 2 888 293 B1

(12) **EUROPEAN PATENT SPECIFICATION**

(45) Date of publication and mention
of the grant of the patent:
**08.05.2019 Bulletin 2019/19**

(51) Int Cl.:
**C08B 11/20** (2006.01)   **A61K 9/28** (2006.01)
**C08B 13/00** (2006.01)

(21) Application number: **13783706.8**

(22) Date of filing: **15.08.2013**

(86) International application number:
**PCT/US2013/055046**

(87) International publication number:
**WO 2014/031422 (27.02.2014 Gazette 2014/09)**

(54) **NOVEL HYDROXYALKYL METHYL CELLULOSE ACETATE SUCCINATES**

NEUARTIGE HYDROXYALKYLMETHYLCELLULOSEACETATSUCCINATE

NOUVEAUX ACÉTATE-SUCCINATES D'HYDROXYALKYLMÉTHYLCELLULOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.08.2012 US 201261692933 P**

(43) Date of publication of application:
**01.07.2015 Bulletin 2015/27**

(73) Proprietor: **Dow Global Technologies LLC**
**Midland, MI 48674 (US)**

(72) Inventors:
• **PETERMANN, Oliver**
 **25462 Rellingen (DE)**
• **SPREHE, Matthias**
 **29664 Walsrode (DE)**
• **GUILLAUDEU, Steven, J.**
 **Midland, MI 48640 (US)**

(74) Representative: **f & e patent**
**Fleischer, Engels & Partner mbB, Patentanwälte**
**Braunsberger Feld 29**
**51429 Bergisch Gladbach (DE)**

(56) References cited:
**WO-A1-2006/082518**

• **RAYMOND CHEN ET AL: "Absolute molecular weight determination of hypromellose acetate succinate by size exclusion chromatography: Use of a multi angle laser light scattering detector and a mixed solvent", JOURNAL OF PHARMACEUTICAL AND BIOMEDICAL ANALYSIS, NEW YORK, NY, US, vol. 56, no. 4, 22 July 2011 (2011-07-22), pages 743-748, XP028269943, ISSN: 0731-7085, DOI: 10.1016/J.JPBA.2011.07.035 [retrieved on 2011-07-30] cited in the application**
• **Anonymous: "Hypromellose Acetate Succinate - Shin-Etsu AQOAT", , 1 October 2005 (2005-10-01), pages 1-20, XP55071998, Retrieved from the Internet: URL:http://www.elementoorganika.ru/files/aqoat.pdf [retrieved on 2013-07-18]**

EP 2 888 293 B1

**Description**

FIELD

This invention concerns novel hydroxyalkyl methyl cellulose acetate succinates, solid dispersions of an active ingredient in such hydroxyalkyl methyl cellulose acetate succinate, as well as liquid compositions, coated dosage forms and capsules comprising such hydroxyalkyl methyl cellulose acetate succinate.

INTRODUCTION

Hydroxyalkyl methyl cellulose acetate succinates, their uses and processes for preparing them are generally known in the art. Various known hydroxyalkyl methyl cellulose acetate succinates are useful as enteric polymers for pharmaceutical dosage forms, such as hydroxypropyl methyl cellulose acetate succinate (HPMCAS). Enteric polymers are those that are resistant to dissolution in the acidic environment of the stomach. Dosage forms coated with such polymers protect the drug from inactivation or degradation in the acidic environment or prevent irritation of the stomach by the drug.

US Patent No. 4,365,060 discloses enterosoluble capsules which are said to have excellent enterosolubility behavior. The enterosoluble capsules are shaped with an ester of a cellulose ether that is esterified with acidic succinyl groups and aliphatic monovalent acyl groups. It is recommended that the cellulose ethers used for esterification have a molecular weight in the range from about 5000 to 200,000 to obtain adequate plasticity.

Wu et al. (Wu S.H. W., Wyatt D.M. and Adams M. W. 1997; Chemistry and applications of cellulosic polymers for enteric coatings of solid dosage forms; in McGinity J. W. (ed.), Aqueous Coatings for Pharmaceutical Dosage Forms, Marcel Dekker, New York, pp. 385 -418) disclose molecular weights of commercially available different grades of HPMCAS. HPMCAS grade AS-L has an $M_w$ of 93,000, an $M_n$ of 46,000 (both measured by gel permeation chromatography method calibrated by polyethylene oxide) and an $M_w/M_n$ of 2.0; HPMCAS grade AS-M has an $M_w$ of 80,000, an Mn of 44,000 and an $M_w/M_n$ of 1.8; and HPMCAS grade AS-H has an $M_w$ of 55,000 an $M_n$ of 33,000 and an $M_w/M_n$ of 1.7. HPMCAS grade AS-L, HPMCAS grade AS-M and HPMCAS grade AS-H which are currently commercially available from Shin-Etsu Chemical Co., Ltd. (Tokyo, Japan), known by the trade name "AQOAT", have higher weight average molecular weights $M_w$, but an increased turbidity in acetone solution. An increased turbidity in acetone solution is an indication of an increased amount of undissolved particles in organic solvents. This is undesirable when a hydroxyalkyl methyl cellulose acetate succinate is subjected to spray-drying because of an increased tendency to clogging devices used for spray-drying, such as spray nozzles. Moreover, an increased amount of undissolved particles in organic solvents is also undesirable when the esterified cellulose ethers are used in transparent films or coatings.

U.S. Patent No. 4,226,981 discloses a process for preparing mixed esters of cellulose ethers, such as hydroxypropyl methyl cellulose acetate succinate (HPMCAS), by esterifying hydroxypropyl methylcellulose with succinic anhydride and acetic anhydride in the presence of an alkali carboxylate as the esterification catalyst and acetic acid as the reaction medium. The cellulose ether as the base material is introduced into the reaction vessel together with about 100 to 2,000 parts by weight of the carboxylic acid as the reaction medium and about 20 to 200 parts by weight of the alkali carboxylate as the catalyst, all being expressed per 100 parts by weight of the cellulose ether, followed by further introduction of predetermined amounts of succinic anhydride and an anhydride of an aliphatic monocarboxylic acid, the resulting mixture being heated at 60 to 110 °C for a period of 2 - 25 hours. In the working examples 250 g of acetic acid and 50 g of sodium acetate are utilized per 50 g of hydroxypropyl methyl cellulose. 15 - 60 g of succinic anhydride and 25 - 80 g of acetic anhydride are added and the reaction mixture is heated at 85 °C with agitation for 3 hours.

European Patent Application EP 0 219 426 discloses a process for producing an enteric-soluble acidic dicarboxylic acid ester of a cellulose ether wherein (a) a cellulose ether having hydroxypropoxyl groups as the ether-forming groups, of which a 2% by weight aqueous solution has a viscosity of at least 5 centipoise at 20 °C, is reacted with (b) a dicarboxylic acid anhydride or a mixture thereof with an anhydride of an aliphatic monocarboxylic acid in the presence of (c) a combination of an alkali metal acetate and acetic acid. EP 0 219 426 shows that the acidic dicarboxylic acid esters produced from cellulose ethers which have a viscosity of at least 6 centipoise provided an enterosoluble film-coating material on tablets which had resistance against a simulated gastric juice. When comparative acidic dicarboxylic acid esters were produced from cellulose ethers having a viscosity of only 3 centipoise, a substantial number of tablets disintegrated in the simulated gastric juice. Acidic dicarboxylic acid esters produced from cellulose ethers of higher viscosity have a higher molecular weight than those produced from cellulose ethers of lower viscosity when comparable process and recipe parameters for producing the acidic dicarboxylic acid esters are applied.

A large number of presently known drugs have a low solubility in water, and thus complex techniques are required to prepare a dosage form. One known method includes dissolving such drug together with a pharmaceutically acceptable water-soluble polymer in an organic solvent that is optionally blended with water, and to spray-dry the solution. Another method is known as melt extrusion, wherein a drug is blended with a pharmaceutically acceptable water-soluble polymer as a powder blend, the powder blend is heated and intensely mixed in the softened or partially or completely melted state and moved towards a die that shapes the melt as strands, films, pellets, tablets or capsules. The pharmaceutically acceptable water-soluble polymer is aimed at reducing the crystallinity of the drug, thereby minimizing the activation energy necessary for the dissolution of the drug, as well as establishing hydrophilic conditions around the drug molecules, thereby improving the solubility of the drug itself to increase its bioavailability, i.e., its *in vivo* absorption by an individual upon ingestion.

International Patent Application WO 2005/115330 discloses hydroxypropyl methyl cellulose acetate succinate (HPMCAS) polymers with a specific combination of substitution levels. The HPMCAS polymer has a degree of substitution of succinoyl groups ($DOS_S$) of at least 0.02, a degree of substitution of acetyl groups ($DOS_{Ac}$) of at least 0.65 and a sum of $DOS_{Ac}$ and $DOS_S$ of at least 0.85. WO 2005/115330 discloses that the increased acetate substitution allows increased solubility of active agents in spray-dried solutions, while the increased succinate substitution increases the solubility of the polymer in aqueous solution.

International Patent Application WO 2011/159626 discloses an active ingredient and HPMCAS having a degree of substitution of methoxy groups ($DS_M$) of $\leq 1.45$, and a combined degree of substitution of acetyl groups ($DS_{Ac}$) and succinoyl groups ($DS_s$) of ($DS_{Ac} + DS_s$) $\geq 1.25$.

However, in view of the large diversity of drugs, it is self-evident that a limited variety of hydroxyalkyl methyl cellulose acetate succinates having a high degree of substitution of acetyl groups and succinoyl groups cannot fulfill all needs. Edgar et al., Cellulose (2007), 14:49-64 "Cellulose esters in drug delivery" state in the conclusion of their survey article: "The fundamental properties of cellulose esters are well-suited to improving drug delivery. ... Much progress has been made in recent years in the application of well-studied cellulose esters to improve drug delivery systems. There is room for much more advancement, particularly by the in-depth study of structure property relationships as they pertain to pharmaceutical applications. Full success in this endeavor will require considerable vision, since the current path to market for novel pharmaceutical excipients is difficult, long, fraught with uncertainty, and expensive."

Accordingly, it is one object of the present invention to provide new hydroxyalkyl methyl cellulose acetate succinates. It is a preferred object of the present invention to provide new hydroxyalkyl methyl cellulose acetate succinates which have a lower turbidity in acetone solution than known hydroxyalkyl methyl cellulose acetate succinates at comparable $M_w$ and comparable degrees of substitution.

## SUMMARY

One aspect of the present invention is a hydroxyalkyl methyl cellulose acetate succinate which a) has from 4.0 to less than 10.0 weight percent of succinoyl groups, b) has a weight average molecular weight $M_w$ of from 80,000 Dalton to 350,000 Dalton, and a z-average molecular weight $M_z$ of from 300,000 to 2,000,000 Dalton, $M_w$ and $M_z$ being measured by SEC-MALLS using as mobile phase a mixture of 40 parts by volume of acetonitrile and 60 parts by volume of aqueous buffer containing 50 mM $NaH_2PO_4$ and 0.1 M $NaNO_3$,c) exhibits a turbidity of up to 41 NTU as a 1.5 weight percent solution in acetone, measured according to USEPA method 180.1, and d) has a viscosity of up to 4.0 mPa·s, measured as a 2.0 wt% solution of the esterified cellulose ether in 0.43 wt% aqueous NaOH at 20 °C according to an Ubbelohde measurement according to DIN 51562-1:1999-01.

Another aspect of the present invention is a hydroxyalkyl methyl cellulose acetate succinate which a) has from 10.0 to 20.0 weight percent of succinoyl groups, b) has a weight average molecular weight $M_w$ of from 80,000 Dalton to 350,000 Dalton and a z-average molecular weight $M_z$ of from 300,000 to 2,000,000 Dalton, $M_w$ and $M_z$ being measured by SEC-MALLS using as mobile phase a mixture of 40 parts by volume of acetonitrile and 60 parts by volume of aqueous buffer containing 50 mM $NaH_2PO_4$ and 0.1 M $NaNO_3$, c) exhibits a turbidity of up to 37 NTU as a 1.5 weight percent solution in acetone, measured according to USEPA method 180.1, and d) has a viscosity of up to 4.0 mPa·s, measured as a 2.0 wt% solution of the esterified cellulose ether in 0.43 wt% aqueous NaOH at 20 °C according to an Ubbelohde measurement according to DIN 51562-1:1999-01.

Another aspect of the present invention is a composition comprising a liquid diluent and at least one hydroxyalkyl methyl cellulose acetate succinate as described above.

Yet another aspect of the present invention is a solid dispersion comprising at least one active ingredient in at least one hydroxyalkyl methyl cellulose acetate succinate as described above.

Yet another aspect of the present invention is a process for producing a solid dispersion which comprises the steps of blending a) at least one hydroxyalkyl methyl cellulose acetate succinate as described above, b) one or more active ingredients and c) one or more optional additives, and subjecting the blend to extrusion.

Yet another aspect of the present invention is a process for producing a solid dispersion which comprises the steps of blending a) at least one hydroxyalkyl methyl cellulose acetate succinate as described above, b) one or more active ingredients, c) one or more optional additives, and d) a liquid diluent to prepare a liquid composition, and removing said liquid diluent.

Yet another aspect of the present invention is a dosage form which is coated with the hydroxyalkyl methyl cellulose acetate succinate as described above.

Yet another aspect of the present invention is a capsule shell which comprises the hydroxyalkyl methyl cellulose acetate succinate as described above.

## DETAILED DESCRIPTION

The hydroxyalkyl methyl cellulose acetate succinate has a cellulose backbone having β-1,4 glycosidically bound D-glucopyranose repeating units, designated as anhydroglucose units in the context of this invention. At least a part of the hydroxyl groups of the anhydroglucose units are substituted by a combination of methoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically, one or two kinds of hydroxyalkoxyl groups are present in the hydroxyalkyl methyl cellulose acetate succinate. Preferably a single kind of hydroxyalkoxyl group, more preferably hydroxypropoxyl, is present.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the esterified cellulose ether. It is to be understood that during the hydroxyalkylation reaction the hydroxyl group of a hydroxyalkoxyl group bound to the cellulose backbone can be further etherified by a methylation agent, and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxy units are covalently bound to each other by ether bonding. Within this definition it is not important whether the terminal hydroxyl group of a hydroxyalkoxyl substituent is further methylated or not; both methylated and non-methylated hydroxyalkoxyl substituents are included for the determination of MS(hydroxyalkoxyl). The hydroxyalkyl methyl cellulose acetate succinate of the invention generally has a molar substitution of hydroxyalkoxyl groups in the range 0.05 to 1.00, preferably 0.08 to 0.90, more preferably 0.12 to 0.70, most preferably 0.15 to 0.60, and particularly 0.20 to 0.50.

The average number of hydroxyl groups substituted by methoxyl groups, per anhydroglucose unit, is designated as the degree of substitution of methoxyl groups, DS(methoxyl). In the above-given definition of DS, the term "hydroxyl groups substituted by methoxyl groups" is to be construed within the present invention to include not only methylated hydroxyl groups directly bound to the carbon atoms of the cellulose backbone, but also methylated hydroxyl groups of hydroxyalkoxyl substituents bound to the cellulose backbone. The esterified cellulose ethers according to this invention preferably have a DS(methoxyl) in the range of 1.0 to 2.5, more preferably from 1.1 to 2.4, most preferably from 1.2 to 2.2 and particularly from 1.6 to 2.05.

Hydroxypropyl methylcellulose acetate succinates (HPMCAS) having the above-mentioned preferred ranges of MS(hydroxypropoxyl) and DS(methoxyl) are preferred. Most preferably, the hydroxypropyl methylcellulose acetate succinates of the present invention have 20 to 26 weight percent of methoxyl groups and 5 to 10 weight percent of hydroxypropoxyl groups, based on the total weight of the hydroxyalkyl methyl cellulose acetate succinate, i.e., including its substituents.

In one embodiment of the present invention the hydroxyalkyl methyl cellulose acetate succinates have from 4.0 to less than 10.0 weight percent, preferably from 4.0 to 8.0 weight percent of succinoyl groups, based on the total weight of the hydroxyalkyl methyl cellulose acetate succinate, i.e., including its substituents. In this embodiment of the present invention the hydroxyalkyl methyl cellulose acetate succinates preferably have from 10.0 to 14.0 weight percent of acetyl groups, based on the total weight of the hydroxyalkyl methyl cellulose acetate succinate. In this embodiment of the present invention the hydroxyalkyl methyl cellulose acetate succinates exhibit a turbidity of up to 41 NTU (nephelometric turbidity units), preferably of up to 40 NTU, more preferably of up to 39 NTU, and most preferably of up to 37 NTU, measured as a 1.5 weight percent solution in acetone.

In another embodiment of the present invention the hydroxyalkyl methyl cellulose acetate succinates have from 10.0 to 20.0 weight percent, preferably from 10.0 to 14.0 or from 14.0 to 18.0 weight percent of succinoyl groups, based on the total weight of the hydroxyalkyl methyl cellulose acetate succinate, i.e., including its substituents. In this embodiment of the present invention the hydroxyalkyl methyl cellulose acetate succinates preferably have from 5.0 to 11.0 weight percent, more preferably from 5.0 to 9.0 or from 7.0 to 11.0 weight percent of acetyl groups, based on the total weight of the hydroxyalkyl methyl cellulose acetate succinate. In this embodiment of the present invention the hydroxyalkyl methyl cellulose acetate succinates exhibit a turbidity of up to 37 NTU, preferably of up to 33 NTU, more preferably of up to 30 NTU, most preferably of up to 28 NTU, and particularly up to 26 NTU, measured as a 1.5 weight percent solution in acetone.

In both embodiments of the invention the turbidity is typically 10 NTU or more, and more typically 11.5 NTU or more. Under optimized conditions the hydroxyalkyl methyl cellulose acetate succinates of the present invention exhibit a turbidity of 10 to 33 NTU or 11.5 to 30 NTU, or 11.5 to 28 NTU, or 11.5 to 26 NTU, or even 11.5 to 20 26 NTU as 1.5 weight percent solution in acetone, measured as indicated below. The hydroxyalkyl methyl cellulose acetate succinates of the present invention exhibit a lower turbidity as 1.5 weight percent solution in acetone than known hydroxyalkyl methyl cellulose acetate succinates of comparable weight average molecular weight and comparable degrees of substitution and satisfies a long-felt need. Hydroxyalkyl methyl cellulose acetate succinates exhibiting a lower turbidity are excellent film-forming polymers or other components of transparent coatings. Moreover, a lower turbidity, measured as a 1.5 weight percent solution in acetone, generally is an indication of a reduced amount of undissolved particles in organic solvents. This is of utmost importance when a hydroxyalkyl methyl cellulose acetate succinate is subjected to spray-drying. A solution comprising a reduced amount of undissolved particles has a reduced tendency to clogging devices used for spray-drying, such as spray nozzles, allowing longer operating periods without interruption for maintenance of the devices used for spray-drying.

1.5 weight % solutions in acetone of the hydroxyalkyl methyl cellulose acetate succinates of the present invention are prepared by mixing the hydroxyalkyl methyl cellulose acetate succinate with acetone and stirring the mixture at room temperature for 24 hours. The turbidity is analyzed with the Turbidimeter 2100AN (wolfram lamp, German catalogue number 47089-00) (Hach Company, Loveland, Colorado, USA). The turbidity is the analysis of the scattered light through a sample cell (diameter: 24mm) and is given in NTUs (nephelometric turbidity units) according to USEPA method 180.1. The analysis is performed against a formazin standard ranging from < 0.1 NTU to 7500 NTU (StablCal™, catalogue number 2659505). A USEPA method 180.1 filter module (catalogue number 3031200) is used. The results given in the examples are the averages of 10 measurements.

The content of the acetate and succinate ester groups is determined according to "Hypromellose Acetate Succinate, United States Pharmacopia and National Formulary, NF 29, pp. 1548-1550". Reported values are corrected for volatiles (determined as described in section "loss on drying" in the above HPMCAS monograph). The method may be used in analogue manner to determine the content of propionyl, butyryl, phthalyl and other ester groups.

The content of hydroxyalkoxyl groups and methoxyl groups is determined in the same manner as described for "Hypromellose", United States Pharmacopeia and National Formulary, USP 35, pp 3467-3469.

The contents of ether and ester groups obtained by the above analyses are converted to DS and MS values of individual substituents according to the formulas below. The formulas may be used in analogue manner to determine the DS and MS of substituents of other cellulose ether esters.

$$
\begin{aligned}
\% \text{ cellulose backbone} \\
= 100 - \left( \%MeO * \frac{M(OCH_3) - M(OH)}{M(OCH_3)} \right) \\
- \left( \%HPO * \frac{M(OCH_2CH(OH)CH_3) - M(OH)}{M(OCH_2CH(OH)CH_3)} \right) \\
- \left( \%Acetyl * \frac{M(COCH_3) - M(H)}{M(COCH_3)} \right) \\
- \left( \%Succinoyl * \frac{M(COC_2H_4COOH) - M(H)}{M(COC_2H_4COOH)} \right)
\end{aligned}
$$

$$
DS(Me) = \frac{\dfrac{\%MeO}{M(OCH_3)}}{\dfrac{\%cellulose\ backbone}{M(AGU)}}
\qquad
MS(HP) = \frac{\dfrac{\%HPO}{M(HPO)}}{\dfrac{\%cellulose\ backbone}{M(AGU)}}
$$

$$
DS(Acetyl) = \frac{\dfrac{\%Acetyl}{M(Acetyl)}}{\dfrac{\%cellulose\ backbone}{M(AGU)}}
\qquad
DS(Succinoyl) = \frac{\dfrac{\%Succinoyl}{M(Succinoyl)}}{\dfrac{\%cellulose\ backbone}{M(AGU)}}
$$

$M(MeO) = M(OCH_3) = 31.03\ Da$ $M(HPO) = M(OCH_2CH(OH)CH_3) = 75.09\ Da$ $M(Acetyl) = M(COCH_3) = 43.04\ Da$

*M(Succinoyl) = M(COC$_2$H$_4$COOH) = 101.08 Da M(AGU) = 162.14 Da M(OH) = 17.008 Da M(H) = 1.008 Da*

By convention, the weight percent is an average weight percentage based on the total weight of the cellulose repeat unit, including all substituents. The content of the methoxyl group is reported based on the mass of the methoxyl group (i.e., -OCH$_3$). The content of the hydroxyalkoxyl group is reported based on the mass of the hydroxyalkoxyl group (i.e., -O-alkylene-OH); such as hydroxypropoxyl (i.e., -O-CH$_2$CH(CH$_3$)-OH). The content of the acetyl groups is reported based on the mass of the acetyl group (-C(O)-CH$_3$). The content of the succinoyl groups is reported based on the mass of succinoyl group (i.e., -C(O)-CH$_2$-CH$_2$-COOH).

The hydroxyalkyl methyl cellulose acetate succinates of the present invention have a weight average molecular weight M$_w$ of from 80,000 to 350,000 Dalton, preferably from 90,000 to 300,000 Dalton, more preferably from 90,000 to 275,000 Dalton, most preferably from 100,000 to 250,000 Dalton, and particularly from 110,000 to 200,000 Dalton..

The hydroxyalkyl methyl cellulose acetate succinates of the present invention preferably have a Polydispersity M$_w$/M$_n$ of at least 1.3, more preferably at least 1.5, most preferably at least, 2.0 or at least 2.3 or at least 2.5. Moreover, the esterified cellulose ethers of the present invention preferably have a Polydispersity of up to 4.1, preferably of up to 3.9, and most preferably of up to 3.7.

The Polydispersity M$_w$/M$_n$ is calculated based on the determination of the weight average molecular weight M$_w$ and the number average molecular weight M$_n$.

The hydroxyalkyl methyl cellulose acetate succinates of the present invention generally have a number average molecular weight M$_n$ of from 23,000 to 150,000 Dalton, preferably from 25,000 to 80,000 Dalton, more preferably from 25,000 to 70,000 Dalton.

The hydroxyalkyl methyl cellulose acetate succinates have a z-average molecular weight, M$_z$, of from 300,000 to 2,000,000 Dalton, more preferably from 500,000 to 1,8000,000 Dalton.

M$_w$, M$_n$ and M$_z$ are measured according to Journal of Pharmaceutical and Biomedical Analysis 56 (2011) 743 using a mixture of 40 parts by volume of acetonitrile and 60 parts by volume of aqueous buffer containing 50 mM NaH$_2$PO$_4$ and 0.1 M NaNO$_3$ as mobile phase. The mobile phase is adjusted to a pH of 8.0. The measurement of M$_w$, M$_n$ and M$_z$ is described in more details in the Examples.

The hydroxyalkyl methyl cellulose acetate succinates of the present invention have a viscosity of up to 4.0 mPa·s, preferably up to 3.6 mPa·s, and more preferably up to 3.2 mPa·s, measured as a 2.0 wt% solution of the hydroxyalkyl methyl cellulose acetate succinate in 0.43 wt% aqueous NaOH at 20 °C. Generally the viscosity is at least 2.4 mPa·s, typically at least 2.5 mPa·s, measured as a 2.0 wt% solution of the hydroxyalkyl methyl cellulose acetate succinate in 0.43 wt% aqueous NaOH at 20 °C. The 2.0 % by weight solution of the hydroxyalkyl methyl cellulose acetate succinate is prepared as described in"Hypromellose Acetate Succinate, United States Pharmacopia and National Formulary, NF 29, pp. 1548-1550", followed by an Ubbelohde viscosity measurement according to DIN 51562-1:1999-01 (January 1999). Hydroxyalkyl methyl cellulose acetate succinates which have a viscosity of up to 5.0 mPa·s, measured as indicated above, can be efficiently produced. It has been found that the viscosity of the hydroxyalkyl methyl cellulose acetate succinate in 0.43 wt% aqueous NaOH substantially corresponds to the viscosity of the cellulose ether which is useful as a starting material for producing the hydroxyalkyl methyl cellulose acetate succinates. A low viscosity cellulose ether used as a starting material allows a good miscibility of the reaction mixture used for producing the hydroxyalkyl methyl cellulose acetate succinates of the present invention resulting in a homogeneous reaction mixture. Moreover, it has also been found that hydroxyalkyl methyl cellulose acetate succinates produced from low viscosity cellulose ethers exhibit a low viscosity in solution when subjected to high shear. This property is highly advantageous when a hydroxyalkyl methyl cellulose acetate succinate is subjected to spray-drying, for example for preparing solid dispersions comprising an active ingredient and a hydroxyalkyl methyl cellulose acetate succinate.

The hydroxyalkyl methyl cellulose acetate succinates of the present invention have a higher M$_w$ than could be expected based on its viscosity, measured as a 2.0 wt% solution of the hydroxyalkyl methyl cellulose acetate succinate in 0.43 wt% aqueous NaOH at 20 °C. Without wanting to be bound by the theory, it is believed that this higher molecular weight is created by hydrophobic/hydrophilic chain association and/or crosslinking reactions. The high molecular weight of the hydroxyalkyl methyl cellulose acetate succinate provides heightened resistance against gastric juice.

Some aspects of the process for producing these hydroxyalkyl methyl cellulose acetate succinates will be described in more general terms below.

For producing a hydroxyalkyl methyl cellulose acetate succinate of the present invention preferably a hydroxyalkyl methyl cellulose is used which has a viscosity of from 2.4 to 5 mPa·s, more preferably from 2.5 to 4 mPa·s, and most preferably from 2.5 to 3.8 mPa·s, measured as a 2 weight-% aqueous solution at 20 °C according to ASTM D2363 - 79 (Reapproved 2006). Hydroxyalkyl methyl celluloses of such viscosity can be obtained by subjecting a hydroxyalkyl methyl cellulose of higher viscosity to a partial depolymerization process. Partial depolymerization processes are well known in the art and described, for example, in European Patent Applications EP 1,141,029; EP 210,917; EP 1,423,433; and US Patent No. 4,316,982. Alternatively, partial depolymerization can be achieved during the production of the hydroxyalkyl methyl celluloses, for example by the presence of oxygen or an oxidizing agent.

The hydroxyalkyl methyl cellulose is reacted with acetic anhydride and succinic anhydride. The two anhydrides may be introduced into the reaction vessel at the same time or separately one after the other. The amount of each anhydride to be introduced into the reaction vessel is determined depending on the desired degree of esterification to be obtained in the final product, usually being 1 to 10 times the stoichiometric amounts of the desired molar degree of substitution of the anhydroglucose units by esterification.

The molar ratio between the acetic anhydride and the anhydroglucose units of the hydroxyalkyl methyl cellulose generally is 0.9 / 1 or more, preferably 1.0 / 1 or more. The molar ratio between the acetic anhydride and the anhydroglucose units of the hydroxyalkyl methyl cellulose generally is 3.0 / 1 or less, preferably 2.7 / 1, more preferably 2.3 / 1.

The molar ratio between the succinic anhydride and the anhydroglucose units of hydroxyalkyl methyl cellulose generally is 0.1 / 1 or more, and preferably 0.13 / 1 or more. The molar ratio between the succinic anhydride and the anhydroglucose units of hydroxyalkyl methyl cellulose generally is 1.0 / 1 or less, and preferably 0.75 / 1 or less.

In the first embodiment of the invention, wherein a hydroxyalkyl methyl cellulose acetate succinate is produced which has from 4.0 to less than 10.0 weight percent of succinoyl groups, the molar ratio between the succinic anhydride and the anhydroglucose units of hydroxyalkyl methyl cellulose preferably is 0.4 or less.

In the second embodiment of the invention, wherein a hydroxyalkyl methyl cellulose acetate succinate is produced which has from 10.0 to 20.0 weight percent of succinoyl groups, the molar ratio between the succinic anhydride and the anhydroglucose units of hydroxyalkyl methyl cellulose preferably is 0.3 or more.

The molar number of anhydroglucose units of the hydroxyalkyl methyl cellulose utilized in the process of the present invention can be determined from the weight of the hydroxyalkyl methyl cellulose used as a starting material, by calculating the average molecular weight of the substituted anhydroglucose units from the DS(methoxyl) and MS(hydroxyalkoxyl).

The esterification of the hydroxyalkyl methyl cellulose is preferably conducted in an aliphatic carboxylic acid as a reaction diluent, such as acetic acid, propionic acid, or butyric acid, most preferably acetic acid. The reaction diluent can comprise minor amounts of other solvents or diluents which are liquid at room temperature and do not react with the hydroxyalkyl methyl cellulose, such as aromatic or aliphatic solvents like benzene, toluene, 1,4-dioxane, or tetrahydrofurane; or halogenated $C_1$-$C_3$ derivatives, like dichloro methane or dichloro methyl ether, but the amount of the aliphatic carboxylic acid is preferably more than 50 percent, more preferably at least 75 percent, and even more preferably at least 90 percent, based on the total weight of the reaction diluent.

Most preferably the reaction diluent consists of an aliphatic carboxylic acid. The molar ratio [aliphatic carboxylic acid / anhydroglucose units of cellulose ether] is generally from [4.9 / 1.0] to [11.5 / 1.0], more preferably from [5.0 / 1.0] to [10.0 / 1.0], more preferably from [5.5 / 1.0] to [9.0 / 1.0], and most preferably from [5.8 / 1.0] to [9.0 / 1.0].

The esterification reaction is generally conducted in the presence of an esterification catalyst, preferably in the presence of an alkali metal carboxylate, such as sodium acetate or potassium acetate. The molar ratio [alkali metal carboxylate / anhydroglucose units of hydroxyalkyl methyl cellulose] is generally from [0.4 / 1.0] to [3.8 / 1.0], and preferably from [1.5 / 1.0] to [3.5/ 1.0].

Especially preferred, the molar ratio [acetic anhydride / succinic anhydride] is from [3.5 / 1] to [8.8 / 1] and the molar ratio [aliphatic carboxylic acid / anhydroglucose units of cellulose ether] is from [4.9 / 1.0] to [11.5 / 1.0].

The reaction mixture is generally heated at 60 °C to 110 °C, preferably at 70 to 100 °C, for a period of time sufficient to complete the reaction, that is, typically from 2 to 25 hours, more typically from 2 to 8 hours. The hydroxyalkyl methyl cellulose as the starting material is not always soluble in the aliphatic carboxylic acid, but can only be dispersed in or swollen by the aliphatic carboxylic acid, especially when the degree of substitution in the hydroxyalkyl methyl cellulose is relatively small. However, the reaction mixture should be thoroughly mixed to provide a homogeneous reaction mixture. As the esterification reaction proceeds, the hydroxyalkyl methyl cellulose under reaction generally dissolves in the reaction diluent.

After completion of the esterification reaction, the reaction product can be precipitated from the reaction mixture in a known manner, for example by contacting with a large volume of water, such as described in U.S. Patent No. 4,226,981, International Patent Application WO 2005/115330 or European Patent Application EP 0 219 426. However, in a preferred embodiment of the invention the reaction product mixture is contacted with an amount of from 5 to 400, preferably from 8 to 300, more preferably from 10 to 100, and most preferably from 12 to 50 weight parts of water per weight part of hydroxyalkyl methyl cellulose used for esterification. The weight ratio [water / reaction product mixture excluding water] is generally from 1/1 to 10/1, preferably from 1.4/1 to 5/1, more preferably from 2/1 to 3 /1. In a preferred embodiment of the invention the combination of water and the reaction product mixture is subjected to a shear rate of at least 800 s$^{-1}$, preferably at least 1500 s$^{-1}$, more preferably at least 3000 s$^{-1}$, and most preferably at least 8000 s$^{-1}$. The shear rate is generally up to 600,000 s$^{-1}$, and typically up to 500,000 s$^{-1}$. Applying such shear rates in the process of the present invention is useful for providing hydroxyalkyl methyl cellulose acetate succinates which are non-tacky and of fine particle size upon precipitation and separation from the reaction product mixture. According to known precipitation processes such non-tacky and fine particles are not achieved. This shear rate can be obtained in a high shear device, such as a high shear mixer, also known as rotor-stator mixer or homogenizer, high shear mill or high shear pump. A high shear device commonly comprises a rotor in combination with a stationary part of the shear device, also referred to as "stationary", such as a stator or housing. The stationary creates a close-clearance gap between the rotor and itself and forms a high-shear zone for materials in this gap. The stationary can include single or multiple rows of openings, gaps or teeth to induce a kind of shear frequency and increased turbulent energy. One metric for the degree or thoroughness of mixing is the shearing force generated by a mixing device with a high tip speed. Fluid undergoes shear when one area of fluid travels with a different velocity relative to an adjacent area. The tip speed of the rotor is a measure of the kinetic energy generated by the rotation according to the formula: Tip speed = rotation rate of rotor x rotor circumference. The shear rate is based on the inverse relationship between the gap distance between the rotor and the stationary part of the shear device which is commonly referred to as the stator or housing. In the case the high shear device is not equipped with a stator, the inner wall of a precipitation vessel serves as a stator. The formula applies: Shear rate = Tip speed / gap distance between outer diameter of rotor and stationary. The high shear device generally runs at a tip speed of at least 4 m/s, preferably at least 8 m/s, more preferably at least 15 m/s, and most preferably at least 30 m/s. The tip speed is generally up to 320 m/s, typically up to 280 m/s.

The dispersed hydroxyalkyl methyl cellulose acetate succinate can subsequently be separated from the remainder of the mixture in a known manner, such as by centrifugation or filtration or upon settling by decantation. The recovered hydroxyalkyl methyl cellulose acetate succinate can be washed with water, preferably by repeatedly slurrying the hydroxyalkyl methyl cellulose acetate succinate in excess water and subjecting the slurry to a shear rate as described further above to remove impurities. The produced hydroxyalkyl methyl cellulose acetate succinate is obtained in the form of a powder upon drying.

Another aspect of the present invention is a composition comprising a liquid diluent and one or more of the above described hydroxyalkyl methyl cellulose acetate succinates. The term "liquid diluent" as used herein means a diluent that is liquid at 25 °C and atmospheric pressure. The diluent can be water or an organic liquid diluent or a mixture of water and an organic liquid diluent. Preferably the amount of the liquid diluent is sufficient to provide sufficient fluidity and processability to the composition for the desired usage, such as spray-drying.

The term "organic liquid diluent" as used herein means an organic solvent or a mixture of two or more organic solvents. Preferred organic liquid diluents are polar organic solvents having one or more heteroatoms, such as oxygen, nitrogen or halogen like chlorine. More preferred organic liquid diluents are alcohols, for example multifunctional alcohols, such as glycerol, or preferably monofunctional alcohols, such as methanol, ethanol, isopropanol or n-propanol; ethers, such as tetrahydrofuran, ketones, such as acetone, methyl ethyl ketone, or methyl isobutyl ketone; acetates, such as ethyl acetate; halogenated hydrocarbons, such as methylene chloride; or nitriles, such as acetonitrile. More preferably the organic liquid diluents have 1 to 6, most preferably 1 to 4 carbon atoms. Specific examples of preferred organic liquid diluents, optionally mixed with minor amounts of water are: methanol, tetrahydrofuran, methylene chloride, a blend of 80 to 95 weight percent of methanol and 20 to 5 weight percent of water, a blend of 80 to 95 weight percent of tetrahydrofuran and 20 to 5 weight percent of water, a blend of 55 to 85 weight percent of acetone and 45 to 15 weight percent of water, a blend of 15 to 85 weight percent of acetone and 85 to 15 weight percent of methanol, a blend of 15 to 85 weight percent of methyl ethyl ketone and 85 to 15 weight percent of methanol, a blend of 30 to 50 weight percent of acrylonitrile and 70 to 50 weight percent of a $C_{1-4}$-monoalcohol, such as methanol, ethanol, isopropylalcohol, or n-propanol; a blend of 30 to 50 weight percent of methanol and 70 to 50 weight percent of tetrahydrofuran or ethyl acetate, or a blend of 70 to 90 weight percent of ethanol and 10 to 30 weight percent of tetrahydrofuran or ethyl acetate.

In one embodiment the composition of the present invention comprises as liquid diluent an organic diluent alone or mixed with a minor amount of water. In this embodiment the composition of the present invention preferably comprises more than 50, more preferably at least 65, and most preferably at least 75 weight percent of an organic liquid diluent and preferably less than 50, more preferably up to 35, and most preferably up to 25 weight percent of water, based on the total weight of the organic liquid diluent and water. This embodiment of the invention is of particularly useful if the present invention comprises an active ingredient of poor water solubility.

In another embodiment the composition of the present invention comprises as liquid diluent water alone or mixed with a minor amount of an organic liquid diluent as described above. In this embodiment the composition of the present invention preferably comprises at least 50, more preferably at least 65, and most preferably at least 75 weight percent of water and preferably up to 50, more preferably up to 35, and most preferably up to 25 weight percent of an organic liquid diluent, based on the total weight of the organic liquid diluent and water. This embodiment of the invention is particularly useful for providing coatings or capsules from aqueous compositions comprising the hydroxyalkyl methyl cellulose acetate succinates of the present invention. When preparing an aqueous solution, it is preferred that at least a portion of the hydroxyalkyl methyl cellulose acetate succinates are in their salt form, preferably in the form of their sodium or potassium salt, more preferably in the form of their sodium salt.

The composition of the present invention comprising a liquid diluent and one or more of the above described hydroxyalkyl methyl cellulose acetate succinates is useful as an excipient system for active ingredients and particularly useful as an intermediate for preparing an excipient system for active ingredients, such as fertilizers, herbicides or pesticides, or biologically active ingredients, such as vitamins, herbals and mineral supplements and drugs. Accordingly, the composition of the present invention preferably comprises one or more active ingredients, most preferably one or more drugs. The term "drug" is conventional, denoting a compound having beneficial prophylactic and/or therapeutic properties when administered to an animal, especially humans. Preferably, the drug is a "low-solubility drug", meaning that the drug has an aqueous solubility at physiologically relevant pH (e.g., pH 1-8) of about 0.5 mg/mL or less. The invention finds greater utility as the aqueous solubility of the drug decreases. Thus, compositions of the present invention are preferred for low-solubility drugs having an aqueous solubility of less than 0.1 mg/mL or less than 0.05 mg/mL or less than 0.02 mg/mL, or even less than 0.01 mg/mL where the aqueous solubility (mg/mL) is the minimum value observed in any physiologically relevant aqueous solution (e.g., those with pH values between 1 and 8) including USP simulated gastric and intestinal buffers. The active ingredient does not need to be a low-solubility active ingredient in order to benefit from this invention, although low-solubility active ingredients represent a preferred class for use with the invention. An active ingredient that exhibits appreciable aqueous solubility in the desired environment of use may have an aqueous solubility up to 1 to 2 mg/mL, or even as high as 20 to 40 mg/mL. Useful low-solubility drugs are listed in the International Patent Application WO 2005/115330, pages 17 - 22.

The composition of the present invention preferably comprises from 1 to 40 weight percent, more preferably from 2.5 to 30 weight percent, most preferably from 5 to 25 weight percent, and particularly from 7 to 20 percent of at least one hydroxyalkyl methyl cellulose acetate succinate as described above, from 40 to 99 weight percent, more preferably from 54.9 to 97.4 weight percent, most preferably from 65 to 94.5 weight percent and particularly from 70 to 92 percent of a liquid diluent described further above, and from 0 to 40 percent, preferably from 0.1 to 40 percent, most preferably from 0.5 to 25 percent, and particularly from 1 to 15 percent of an active ingredient, based on the total weight of the composition.

In one aspect of the invention the composition comprising at least one hydroxyalkyl methyl cellulose acetate succinate as described above, one or more active ingredients and optionally one or more adjuvants can be used in liquid form, for example in the form of a suspension, a slurry, a sprayable composition, or a syrup. The liquid composition is useful, e.g., for oral, ocular, topical, rectal or nasal applications. The liquid diluent should generally be pharmaceutically acceptable, such as ethanol or glycerol, optionally mixed with water as described above.

In another aspect of the invention the liquid composition of the present invention is used for producing a solid dispersion comprising at least one active ingredient, such as a drug described further above, at least one hydroxyalkyl methyl cellulose acetate succinate as described above and optionally one or more adjuvants. The solid dispersion is produced by removing the liquid diluent from the composition.

One method of removing the liquid diluent from the liquid composition is by casting the liquid composition into a film or a capsule or by applying the liquid composition onto a solid carrier that in turn may comprise an active ingredient. A preferred method of producing the solid dispersion is by spray-drying. The term "spray-drying" refers to processes involving breaking up liquid mixtures into small droplets (atomization) and rapidly removing solvent from the mixture in a spray-drying apparatus where there is a strong driving force for evaporation of solvent from the droplets. Spray-drying processes and spray-drying equipment are described generally in Perry's Chemical Engineers' Handbook, pages 20-54 to 20-57 (Sixth Edition 1984). More details on spray-drying processes and equipment are reviewed by Marshall, "Atomization and Spray-Drying," 50 Chem. Eng. Prog. Monogr. Series 2 (1954), and Masters, Spray Drying Handbook (Fourth Edition 1985). A useful spray-drying process is described in the International Patent Application WO 2005/115330, page 34, line 7 - page 35, line 25.

Alternatively, the solid dispersion of the present invention may be prepared by i) blending a) at least one hydroxyalkyl methyl cellulose acetate succinate defined above, b) one or more active ingredients and c) one or more optional additives, and ii) subjecting the blend to extrusion. The term "extrusion" as used herein includes processes known as injection molding, melt casting and compression molding. Techniques extruding, preferably for melt-extruding compositions comprising an active ingredient such as a drug are known and described by Joerg Breitenbach, Melt extrusion: from process to drug delivery technology, European Journal of Pharmaceutics and Biopharmaceutics 54 (2002) 107-117 or in European Patent Application EP 0 872 233. The above-mentioned components a), b) and optionally c) are preferably mixed in the form of particles, more preferably in powdered form. The components a), b) and optionally c) may be pre-mixed before feeding the blend into a device utilized for extrusion. Useful devices for extrusion, specifically useful extruders, are known in the art. Alternatively, the components a), b) and optionally c) may be fed separately into the extruder and blended in the device before or during a heating step. Preferably components a), b) and optionally c) are pre-blended in an extruder feeder and fed from there into the extruder. The composition or the component(s) that has or have been fed into an extruder are passed through a heated area of the extruder at a temperature which will melt or soften the composition or at least one or more components thereof to form a blend throughout which the active ingredient is dispersed. The blend is subjected to extrusion, preferably melt-extrusion, and caused to exit the extruder. Typical extrusion temperatures are from 50 to 210 °C, preferably from 70 to 200 °C, more preferably from 90 to 190°C, as determined by the setting for the extruder heating zone(s). An operating temperature range should be selected that will minimize the degradation or decomposition of the active ingredient and other components of the composition during processing. Single or multiple screw extruders, preferably twin screw extruders, can be used in the extrusion process. The molten or softened mixture obtained in the extruder is forced through one or more exit openings, such as one or more nozzles or dies. The molten or softened mixture then exits via a die or other such element having one or a plurality of openings, at which time, the extruded blend (now called the extrudate) begins to harden. Since the extrudate is still in a softened state upon exiting the die, the extrudate may be easily shaped, molded, chopped, spheronized into beads, cut into strands, tabletted or otherwise processed to the desired physical form. The extrudate can optionally be cooled to hardening and ground into a powdered form.

The solid dispersion of the present invention preferably comprises from 20 to 99.9 percent, more preferably from 30 to 98 percent, and most preferably from 60 to 95 percent of a hydroxyalkyl methyl cellulose acetate succinate a) as described above, and preferably from 0.1 to 80 percent, more preferably from 2 to 70 percent, and most preferably from 5 to 40 percent of an active ingredient b), based on the total weight of the hydroxyalkyl methyl cellulose acetate succinate a) and the active ingredient b). The combined amount of the hydroxyalkyl methyl cellulose acetate succinate a) and the active ingredient b) is preferably at least 70 percent, more preferably at least 80 percent, and most preferably at least 90 percent, based on the total weight of the solid dispersion. The remaining amount, if any, are one or more of the adjuvants c) as described below. The solid dispersion can comprise one or more of the hydroxyalkyl methyl cellulose acetate succinates a), one or more of the active ingredients b), and optionally one or more of the adjuvants c), however their total amount is generally within the above-mentioned ranges.

Once the solid dispersion comprising at least one active ingredient in at least one hydroxyalkyl methyl cellulose acetate succinate has been formed, several processing operations can be used to facilitate incorporation of the dispersion into a dosage form. These processing operations include drying, granulation, and milling. The inclusion of optional adjuvants in the solid dispersion may be useful in order to formulate the composition into dosage forms. The solid dispersion of the present invention may be in various forms, such as, e.g. in the form of strands, pellets, granules, pills, tablets, caplets, microparticles, fillings of capsules or injection molded capsules or in the form of a powder, film, paste, cream, suspension or slurry.

The amount of the active ingredient in the dosage form is generally is at least 0.1 percent, preferably at least 1 percent, more preferably at least 3 percent, most preferably at least 5 percent and generally up to 70 percent, preferably up to 50 percent, more preferably up to 30 percent, most preferably up to 25 percent, based on the total weight of the dosage form.

In another aspect of the invention the composition of the present invention comprising a liquid diluent and one or more of the above described hydroxyalkyl methyl cellulose acetate succinate may be used for coating dosage forms, such as tablets, granules, pellets, caplets, lozenges, suppositories, pessaries or implantable dosage forms, to form a coated composition. If the composition of the present invention comprises an active ingredient, such as a drug, drug layering can be achieved, i.e., the dosage form and the coating may comprise different active ingredients for different end-uses and/or having different release kinetics.

In yet another aspect of the invention the composition of the present invention comprising a liquid diluent and one or more of the above described hydroxyalkyl methyl cellulose acetate succinates may be used for the manufacture of capsules in a process which comprises the step of contacting the liquid composition with dipping pins.

The liquid composition and the solid dispersion of the present invention may further comprise optional additives, such as coloring agents, pigments, opacifiers, flavor and taste improvers, antioxidants, and any combination thereof. Optional additives are preferably pharmaceutically acceptable. Useful amounts and types of one or more optional adjuvants are generally known in the art and depend on the intended end-use of the liquid composition or the solid dispersion of the present invention.

The following examples are for illustrative purposes only and are not intended to limit the scope of the present invention.

EXAMPLES

Unless otherwise mentioned, all parts and percentages are by weight. In the Examples the following test procedures are used.

Content of ether and ester groups

The content of ether groups in the esterified cellulose ether is determined in the same manner as described for "Hypromellose", United States Pharmacopeia and National Formulary, USP 35, pp 3467-3469.

The ester substitution with acetyl groups ($-CO-CH_3$) and the ester substitution with succinoyl groups ($-CO-CH_2-CH_2-COOH$) are determined according to Hypromellose Acetate Succinate, United States Pharmacopia and National Formulary, NF 29, pp. 1548-1550". Reported values for ester substitution are corrected for volatiles (determined as described in section "loss on drying" in the above HPMCAS monograph).

Determination of the Turbidity

1.5 weight % solutions in acetone of the hydroxyalkyl methyl cellulose acetate succinates of the present invention are prepared by mixing the hydroxyalkyl methyl cellulose acetate succinate with acetone and stirring the mixture at room temperature for 24 hours. The turbidity is analyzed with the Turbidimeter 2100AN (wolfram lamp, German catalogue number 47089-00) (Hach Company, Loveland, Colorado, USA). The turbidity is the analysis of the scattered light through a sample cell (diameter: 24mm) and is given in NTUs (nephelometric turbidity units) according to USEPA method 180.1. The analysis is performed against a formazin standard ranging from < 0.1 NTU to 7500 NTU (StablCal™, catalogue number 2659505). A USEPA method 180.1 filter module (catalogue number 3031200) is used. The results given in the examples are the averages of 10 measurements.

Determination of $M_w$, $M_n$ and $M_z$

Mw, Mn and Mz are measured according to Journal of Pharmaceutical and Biomedical Analysis 56 (2011) 743 unless stated otherwise. The mobile phase was a mixture of 40 parts by volume of acetonitrile and 60 parts by volume of aqueous buffer containing 50 mM NaH2PO4 and 0.1 M NaNO3. The mobile phase was adjusted to a pH of 8.0. Solutions of the cellulose ether esters were filtered into a HPLC vial through a syringe filter of 0.45 $\mu$m pore size.
More specifically, the utilized Chemicals and solvents were:
Polyethylene oxide standard materials (abbreviated as PEOX 20 K and PEOX 30 K) were purchased from Agilent Technologies, Inc. Palo Alto, CA, catalog number PL2083-1005 and PL2083-2005.
Acetonitrile (HPLC grade ≥ 99.9 %, CHROMASOL plus), catalog number 34998, sodium hydroxide (semiconductor grade, 99.99 %, trace metal base), catalog number 306576, water (HPLC grade, CHROMASOLV Plus) catalog number 34877 and sodium nitrate (99,995 %, trace metal base) catalog number 229938 were purchased from Sigma-Aldrich, Switzerland.
Sodium dihydrogen phosphate (≥ 99.999 % TraceSelect) catalog number 71492.was purchased from FLUKA, Switzerland.
The normalization solution of PEOX20 K at 5 mg/mL, the standard solution of PEOX30 K at 2 mg/mL, and the sample solution of HPMCAS at 2 mg/mL were prepared by adding a weighed amount of polymer into a vial and dissolving it with a measured volume of mobile phase. All solutions were allowed to dissolve at room temperature in the capped vial for 24 h with stirring using a PTFE-coated magnetic stirring bar.
The normalization solution (PEOX 20k, single preparation, N) and the standard solution (PEOX30 K, double preparation, S1 and S2) were filtered into a HPLC vial through a syringe filter of 0.02 $\mu$m pore size and 25 mm diameter (Whatman Anatop 25, catalog number 6809-2002), Whatman.
The test sample solution (HPMCAS, prepared in duplicate, T1, T2) and a laboratory standard (HPMCAS, single preparation, LS) were filtered into a HPLC vial through a syringe filter of 0.45 $\mu$m pore size (Nylon, e.g. Acrodisc 13 mm VWR catalog number 514-4010).
Chromatographic condition and run sequence were conducted as described by Chen, R. et al.; Journal of Pharmaceutical and Biomedical Analysis 56 (2011) 743- 748). The SEC-MALLS instrument set-up included a HP1100 HPLC system from Agilent Technologies, Inc. Palo Alto, CA; a DAWN Heleos II 18 angle laser light scattering detector and a OPTILAB rex refractive index detector, both from Wyatt Technologies, Inc. Santa Barbara, CA. The analytical size exclusion column (TSK-GEL® GMPWXL, 300 × 7.8 mm) was purchased from Tosoh Bioscience. Both the OPTILAB and the DAWN were operated at 35 °C. The analytical SEC column was operated at room temperature (24 ± 5 °C). The mobile phase was a mixture of 40 volume parts of acetonitrile and 60 volume parts of aqueous buffer containing 50 mM NaH2PO4 and 0.1 M NaNO3 prepared as follows:

Aqueous buffer: 7.20 g of sodium dihydrogen phosphate and 10.2 g of sodium nitrate were added to 1.2 L purified water in a clean 2 L glass bottle under stirring until dissolution.
Mobile phase: 800 mL of acetonitrile were added to 1.2 L of the aqueous buffer prepared above, and stirred until a good mixture was achieved and the temperature equilibrated to ambient temperature.

The mobile phase was pH adjusted to 8.0 with 10M NaOH and filtered through a 0.2 m nylon membrane filter. The flow rate was 0.5 mL/min with in-line degassing. The injection volume was 100 $\mu$L and the analysis time was 35 min.
The MALLS data were collected and processed by Wyatt ASTRA software (version 5.3.4.20) using dn/dc value (refractive index increment) of 0.120 mL/g for HPMCAS. The light scattering signals of detector Nos. 1-4, 17, and 18) were not used in the molecular weight calculation. A representative chromatographic run sequence is given below: B, N, LS, S1 (5x), S2, T1 (2x), T2 (2x), T3 (2x), T4 (2x), S2, T5(2x), etc., S2, LS, W, where, B represents blank injection of mobile phase, N1 represents normalization solution; LS represents a laboratory standard HPMCAS; S1 and S2 represent standard solutions one and two, respectively; T1, T2, T3, T4, and T5 represent test sample solutions and W represents water injection. (2x) and (5x) denote the number of injections of the same solution.

Both the OPTILAB and the DAWN were calibrated periodically according to the manufacturer's recommended procedures and frequency. A 100 μL injection of a 5 mg/mL polyethylene oxide standard (PEOX20 K) was employed for normalizing all angle light scattering detectors relative to 90° detector for each run sequence.

Use of this mono-dispersed polymer standard also enabled the volume delay between the OPTILAB and the DAWN to be determined, permitting proper alignment of the light scattering signals to the refractive index signal. This is necessary for the calculation of the weight-averaged molecular weight (Mw) for each data slice.

Production of HPMCAS of Examples 1-10

Glacial acetic acid, acetic anhydride, a hydroxypropyl methylcellulose (HPMC), succinic anhydride and sodium acetate (water free) were introduced in the amounts listed in Table 1 below into a reaction vessel under thorough stirring.

The HPMC had a methoxyl and hydroxypropoxyl substitution as listed in Table 2 below and a viscosity of about 3 mPa·s, measured as a 2 % solution in water at 20 °C according to ASTM D2363 - 79 (Reapproved 2006). The HPMC is commercially available from The Dow Chemical Company as Methocel E3 LV Premium cellulose ether.

The mixture was heated at 85° C with agitation for 3 or 3.5 hours, as listed in Table 1 below, to effect esterification. x L of water was added to the reactor under stirring to precipitate the HPMCAS. The precipitated product was removed from the reactor and washed with y L of water by applying high shear mixing using an Ultra-Turrax stirrer S50-G45 running at 5200 rpm. The numbers of water x and y are listed in Table 1 below. The product was isolated by filtration and dried at 50°C. The product was then thoroughly washed by repeatedly slurrying the product in excess water using the Ultra-Turrax stirrer S50-G45 and isolating the product by filtration. The washed product was dried again at 50°C.

Production of HPMCAS of Comparative Examples A and B

The production of HPMCAS according to Comparative Examples A and B was carried out as in Examples 1 to 10, except that the weight ratios of glacial acetic acid, acetic anhydride, a hydroxypropyl methylcellulose (HPMC), succinic anhydride and sodium acetate (water free) were used as disclosed in Example 2 of European Patent Application EP 0219 426 A2. The used amounts are listed in Table 1 below.

The HPMC used in Comparative Examples A and B respectively had a viscosity of about 6 mPa·s and about 3 mPa·s respectively, measured as a 2 % solution in water at 20 °C according to ASTM D2363 - 79 (Reapproved 2006). Each HPMC contained about 10 % by weight of hydroxypropoxyl groups and about 29 % by weight of methoxyl groups. These HPMC's are commercially available from The Dow Chemical Company as Methocel E6 LV Premium cellulose ether and Methocel E3 LV Premium cellulose ether respectively.

The mixture was heated at 85° C with agitation for 3.5 hours to effect esterification. x L of water was added to the reactor under stirring to precipitate the HPMCAS. The precipitated product was removed from the reactor and washed with y L of water by applying high shear mixing using an Ultra-Turrax stirrer S50-G45 running at 5200 rpm. The numbers of water x and y are listed in Table 1 below. The product was isolated by filtration and dried at 55°C for 12h. The product was then thoroughly washed and dried again as described in Examples 1 - 10.

Production of Comparative Example C

The production of HPMCAS according to Comparative Example C was carried out as in Examples 1 to 10, except that the weight ratios of glacial acetic acid, acetic anhydride, HPMC, succinic anhydride and sodium acetate (water free) were used as disclosed in Comparative Example 3 of US Patent No. 5,776,501. The HPMC used in Comparative Example 3 of US Patent No. 5,776,501 had a viscosity of 8.9 mPa·s, measured as a 2 % solution in water. However, to avoid that differences in HPMC viscosity have an impact on the molecular weight of the HPMCAS, the same HPMC was used in Comparative Example C as in Examples 1 to 10.

The used amounts are listed in Table 1 below. The mixture was heated at 85° C with agitation for 5 hours to effect esterification. 252.86 g of water was added to the reactor under stirring, followed by addition of 70.71 g concentrated hydrochloric acid (concentration of 37 wt-%). The precipitated product was obtained by adding the reaction mixture to 3.0L of water under stirring (200 rpm). The crude product was washed with 11L of water by applying high shear mixing using an Ultra-Turrax stirrer S50-G45 running at 5200 rpm. The product was isolated by filtration and dried at 55°C for 12 h. The product was then thoroughly washed and dried again as described in Examples 1 - 10.

Repetition of Comparative Examples A - C

The obtained ester substitutions % acetyl and % succinoyl in Comparative Examples A and B were significantly different from those disclosed in Example 2 of European Patent Application EP 0219 426 A2. In Comparative Example C the obtained ester substitutions % acetyl and % succinoyl matched reasonably well with the ester substitutions reported in Comparative Example 3 of US Patent No. 5,776,501.

Therefore, Comparative Examples A - C were repeated. The obtained ester substitutions % acetyl and % succinoyl in the repeated set of Comparative Examples A - C were substantially the same as in the first set of Comparative Examples A - C. The obtained ester substitutions % acetyl and % succinoyl and the Mw, Mn and Mz in Tables 2 and 3 show the average of the two sets of Comparative Examples A, B, and C.

Production of Comparative Examples D - H

The production of HPMCAS according to Comparative Examples D - H was carried out as in Examples 1 to 10, except that the weight ratios of glacial acetic acid, acetic anhydride, HPMC, succinic anhydride and sodium acetate (water free) were used as disclosed in Example 1 of US Patent No. 4,226,981, Table I, Sample Nos. 1 - 5. The used amounts are listed in Table 1 below. The US Patent No. 4,226,981 is silent about the viscosity of the used HPMC. To avoid that differences in HPMC viscosity have an impact on the molecular weight of the HPMCAS, the same HPMC was used in Comparative Example D - H as in Examples 1 - 10. x L of water was added to the reactor under stirring to precipitate the HPMCAS. The precipitated product was removed from the reactor and washed with y L of water by applying high shear mixing using an Ultra-Turrax stirrer S50-G45 running at 5200 rpm. The numbers of water x and y are listed in Table 1 below. The product was then thoroughly washed and dried again as described in Examples 1 - 10.

Production of Comparative Examples I and J

The production of HPMCAS according to Comparative Example I and J was carried out as in Examples 1 to 10, except that the weight ratios of glacial acetic acid, acetic anhydride, HPMC, succinic anhydride and sodium acetate (water free) were used as disclosed International Patent Application WO 2005/115330, pages 51 and 52, polymers 1 and 3. The product was obtained, separated and washed as described in International Patent Application WO 2005/115330. The reaction mixture was quenched into 2.4L of water, precipitating the polymer. An additional 1L of water was used to complete the precipitation for example I only. The polymer was then isolated and washed with 3x 300 mL of water. Then the polymer was dissolved in 600 mL of acetone and again precipitated in 2.4L of water. To complete precipitation another 1L of water was added. The product was then thoroughly washed and dried again as described in Examples 1 - 10.

Comparative Examples K to M

As disclosed in International Patent Application WO 2011/159626 on pages 1 and 2, HPMCAS is currently commercially available from Shin-Etsu Chemical Co., Ltd. (Tokyo, Japan), known by the trade name "AQOAT". Shin-Etsu manufactures three grades of AQOAT polymers that have different combinations of substituent levels to provide enteric protection at various pH levels, AS-L, AS-M, and AS-H, typically followed by the designation "F" for fine or "G", such as AS-LF or AS-LG. Their sales specifications are listed below.

Properties of AQOAT polymers as listed in WO 2011/159626

| Designation of analyzed commercial samples: Comparative Example | K | L | M |
|---|---|---|---|
| Substituent content | Published Composition of AQOAT polymers (wt %) | | |
| | L-Grade | M-Grade | H-Grade |
| Methoxyl | 20.0 - 24.0 | 21-0 - 25.0 | 22.0 - 26.0 |
| Hydroxypropoxyl | 5.0 - 9.0 | 5.0 - 9.0 | 6.0 - 10.0 |
| Acetyl | 5.0 - 9.0 | 7.0 - 11.0 | 10.0 - 14.0 |
| Succinoyl | 14.0 - 18.0 | 10 - 14 | 4.0 - 8.0 |

Samples of the commercially available materials were analyzed as described further above.

Comparative Examples N, O-1, O-2, P-1 and P-2

HPMCAS samples were produced as described on pages 34 and 35 of WO 2011/159626. In Comparative Example N the recipe for HPMCAS-K(1) was exactly repeated. In Comparative Examples O-1 and O-2 the recipe for HPMCAS-K(2) and in Comparative Examples P-1 and P-2 the recipe for HPMCAS-K(3) were exactly repeated. Comparative Examples O and P were each conducted twice and reported as O-1, O-2, P-1 and P-2 respectively since the results in Comparative Examples O-1 and P-1 for $DOS_{Ac}$ and $DOS_s$ deviated from the results reported in WO 2011/159626 for HPMCAS-K(2) and HPMCAS-K(3).The properties of the HPMCAS produced according to Examples 1 - 10 and comparative Examples A - J, N, O-1, O-2, P-1 and P-2 and the properties of the commercially available Comparative Examples K to M are listed in Table 2 below. In Table 2 below the abbreviations have the following meanings:

$DS_M$ = DS(methoxyl): degree of substitution with methoxyl groups;
$MS_{HP}$ = MS(hydroxypropoxyl): molar subst. with hydroxypropoxyl groups;
$DOS_{Ac}$: degree of substitution of acetyl groups;
$DOS_s$: degree of substitution of succinoyl groups.

Table 1

| Example | HPMC* | | acetic acid | | Succinic anhydride | | Acetic anhydride | | Sodium acetate | | Heating at 85 °C | x L of water | y L of water |
|---|---|---|---|---|---|---|---|---|---|---|---|---|---|
| | g | Mol | g | mol/mol HPMC | g | mol/mol HPMC | g | mol/mol HPMC | g | mol/mol HPMC | hours | | |
| 1 | 187.5 | 0.93 | 500 | 9.0 | 52.5 | 0.57 | 243.8 | 2.69 | 187.5 | 2.47 | 3.5 | 1.8 | 35 |
| 2 | 195 | 0.97 | 442 | 7.6 | 54.6 | 0.57 | 253.5 | 2.69 | 195.0 | 2.47 | 3.5 | 1.8 | 35 |
| 3 | 195 | 0.97 | 440 | 7.6 | 40.0 | 0.42 | 200 | 2.12 | 120 | 1.52 | 3 | 1.8 | 16 |
| 4 | 195 | 0.97 | 440 | 7.6 | 40.0 | 0.42 | 200 | 2.12 | 195 | 2.47 | 3 | 1.8 | 16 |
| 5 | 230 | 1.14 | 521 | 7.6 | 64.4 | 0.57 | 299.0 | 2.69 | 259.5 | 2.78 | 3 | 2.1 | 30.5 |
| 6 | 230 | 1.14 | 521 | 7.6 | 64.4 | 0.57 | 299.0 | 2.69 | 283.1 | 3.04 | 3 | 2.1 | 34 |
| 7 | 230 | 1.14 | 521 | 7.6 | 64.4 | 0.57 | 299.0 | 2.69 | 306.7 | 3.29 | 3 | 2.1 | 15.5 |
| 8 | 150 | 0.74 | 340 | 7.6 | 42.0 | 0.57 | 195.0 | 2.69 | 215.4 | 3.54 | 3 | 1.4 | 36.5 |
| 9 | 230 | 1.14 | 440 | 6.5 | 22.0 | 0.19 | 200 | 1.8 | 230 | 2.47 | 3 | 2.3 | 13 |
| 10 | 230 | 1.14 | 335 | 4.9 | 17.4 | 0.15 | 126.5 | 1.14 | 49.3 | 0.53 | 3.5 | 2.3 | 15 |
| A[1] | 100 | 0.49 | 300 | 10.1 | 25 | 0.51 | 38 | 0.78 | 80 | 1.97 | 3.5 | 1.2 | 12 |
| B[2] | 200 | 0.96 | 600 | 10.2 | 50 | 0.51 | 76 | 0.78 | 160 | 1.97 | 3.5 | 2.4 | 11 |
| C | 150 | 0.74 | 450 | 10.1 | 35.8 | 0.48 | 57.43 | 0.79 | 59.57 | 0.98 | 5 | 3.0 | 11 |
| D | 115 | 0.57 | 575 | 16.9 | 138 | 2.44 | 57.5 | 1.03 | 115 | 2.47 | 3 | 2.3 | 10 |
| E | 115 | 0.57 | 575 | 16.9 | 92 | 1.63 | 57.5 | 1.03 | 115 | 2.47 | 3 | 2.3 | 10 |
| F | 115 | 0.57 | 575 | 16.9 | 57.5 | 1.02 | 115 | 2.06 | 115 | 2.47 | 3 | 2.3 | 10 |
| G | 115 | 0.57 | 575 | 16.9 | 46 | 0.81 | 138 | 2.48 | 115 | 2.47 | 3 | 2.3 | 10 |
| H | 115 | 0.57 | 575 | 16.9 | 34.5 | 0.61 | 184 | 3.30 | 115 | 2.47 | 3 | 2.3 | 10 |
| I | 80 | 0.40 | 420 | 17.7 | 18.9 | 0.48 | 640.2 | 16.53 | 40.43 | 1.25 | 21.75 | See Comp. Examples I and J above | |
| J | 80 | 0.40 | 420 | 17.7 | 13.2 | 0.33 | 432.8 | 11.17 | 40.43 | 1.25 | 21.75 | | |

* calculated on the dried basis
1) Comparative Example A: HPMC of 6 mPa·s
2) Comparative Example B: HPMC of 3 mPa·s

Table 2

| (Comp.) Example | Molecular weight (kDA) | | | | | 2% viscosity* (mPa·s) | Ether Substitution | | Ester substitution | | Ether Substitution | | Ester substitution | | Turbidity 1.5 wt. % in acetone |
|---|---|---|---|---|---|---|---|---|---|---|---|---|---|---|---|
| | Mw | Mn | Mw / Mn | Mz | Recovery Rate (%) | | Methoxyl (%) | HydroxyPropoxyl, % | Acetyl (%) | Succinoyl (%) | $DS_M$ | $MS_{HP}$ | $DOS_{Ac}$ | $DOS_s$ | |
| 1 | 113 | 31 | 3.6 | 1320 | 97 | 2.61 | 22.8 | 7.5 | 10.6 | 12.4 | 1.94 | 0.26 | 0.65 | 0.32 | 23.3 |
| 2 | 139 | 36 | 3.9 | 1540 | 96 | 2.61 | 22.7 | 7.5 | 11.0 | 12.1 | 1.94 | 0.26 | 0.68 | 0.32 | 24.9 |
| 3 | 95 | 28 | 3.4 | 703 | 100 | 2.66 | 23.1 | 7.5 | 9.7 | 11.7 | 1.92 | 0.26 | 0.58 | 0.30 | 13.4 |
| 4 | 144 | 38 | 3.8 | 907 | 98 | 2.64 | 22.9 | 7.5 | 9.9 | 12.3 | 1.93 | 0.26 | 0.60 | 0.32 | 12.4 |
| 5 | 195 | 57 | 3.4 | 1073 | 98 | 2.65 | 22.7 | 7.2 | 10.7 | 12.3 | 1.93 | 0.25 | 0.65 | 0.32 | 18.8 |
| 6 | 248 | 75 | 3.3 | 1235 | 98 | 2.70 | 22.6 | 7.2 | 10.9 | 12.4 | 1.93 | 0.25 | 0.67 | 0.32 | 15.7 |
| 7 | 266 | 81 | 3.3 | 1297 | 97 | 2.68 | 22.4 | 7.2 | 10.9 | 12.5 | 1.91 | 0.25 | 0.67 | 0.33 | 14.4 |
| 8 | 305 | 97 | 3.1 | 1414 | 97 | 2.71 | 22.5 | 7.2 | 10.8 | 12.4 | 1.91 | 0.25 | 0.66 | 0.32 | 17.5 |
| 9 | 175 | 49 | 3.6 | 1109 | 95 | 2.71 | 23.7 | 7.6 | 12.8 | 6.5 | 1.92 | 0.25 | 0.75 | 0.16 | 23.4 |
| 10 | 142 | 44 | 3.2 | 1018 | 101 | 2.97 | 24.1 | 7.8 | 11.5 | 5.6 | 1.90 | 0.25 | 0.65 | 0.14 | 36.2 |
| A[1] | 270 | 87 | 3.1 | 1060 | 101 | 4.58 | 21.9 | 7.2 | 5.6 | 16.8 | 1.83 | 0.25 | 0.34 | 0.43 | 13.6 |
| B[2] | 65 | 26 | 2.5 | 329 | 95 | 2.89 | 22.9 | 7.3 | 5.7 | 16.0 | 1.91 | 0.25 | 0.34 | 0.41 | 13.1 |
| C | 53 | 23 | 2.3 | 342 | 103 | 2.90 | 23.7 | 7.6 | 5.8 | 14.7 | 1.96 | 0.26 | 0.35 | 0.37 | 4.6 |
| D | 37 | 25 | 1,5 | 56 | 102 | 3.06 | 20.1 | 6.4 | 2.6 | 25.0 | 1.79 | 0.24 | 0.17 | 0.68 | 3.0 |
| E | 38 | 25 | 1.5 | 65 | 104 | 2.89 | 21.6 | 6.6 | 3.3 | 22.7 | 1.90 | 0.24 | 0.21 | 0.61 | 2.2 |
| F | 41 | 23 | 1.8 | 111 | 98 | 2.92 | 21.6 | 7.0 | 6.2 | 15.8 | 1.79 | 0.24 | 0.37 | 0.40 | 3.1 |
| G | 40 | 22 | 1.8 | 123 | 101 | 2.98 | 23.1 | 7.2 | 7.8 | 13.5 | 1.92 | 0.25 | 0.47 | 0.34 | 3.1 |
| H | 36 | 20 | 1.8 | 119 | 101 | 3.73 | 23.5 | 7.6 | 9.7 | 9.6 | 1.90 | 0.25 | 0.57 | 0.24 | 4.4 |
| I | 51 | 23 | 2.2 | 462 | 105 | 2.86 | 22.3 | 7.4 | 11.7 | 11.7 | 1.90 | 0.26 | 0.72 | 0.31 | 1.3 |
| J | 54 | 22 | 2.5 | 1158 | 98 | 2.86 | 22.6 | 7.1 | 12.6 | 9.1 | 1.87 | 0.24 | 0.75 | 0.23 | 2.0 |
| K | 153 | 33 | 4.6 | 889 | 100 | 3.00 | 22.5 | 7.0 | 8.1 | 14.7 | 1.90 | 0.24 | 0.49 | 0.38 | 39.4 |
| L | 104 | 25 | 4.2 | 512 | 100 | 3.00 | 23.2 | 7.2 | 9.4 | 10.8 | 1.89 | 0.24 | 0.55 | 0.27 | 43.7 |
| M | 137 | 29 | 4.7 | 821 | 97 | 3.00 | 23.6 | 7.2 | 11.5 | 7.8 | 1.90 | 0.24 | 0.67 | 0.19 | 42.4 |

(continued)

| Table 2 | Molecular weight (kDA) | | | | | 2% viscosity* (mPa·s) | Ether Substitution | | Ester substitution | | Ether Substitution | | Ester substitution | | Turbidity 1.5 wt.% in acetone |
|---|---|---|---|---|---|---|---|---|---|---|---|---|---|---|---|
| (Comp.) Example | Mw | Mn | Mw / Mn | Mz | Recovery Rate (%) | | Methoxyl (%) | HydroxyPropoxyl, % | Acetyl (%) | Succinoyl (%) | $DS_M$ | $MS_{HP}$ | $DOS_{Ac}$ | $DOS_s$ | |
| N | 427 | 94 | 4.5 | 2592 | 99 | 2.06 | 15.7 | 6.2 | 12.1 | 20.8 | 1.47 | 0.24 | 0.82 | 0.60 | ns[3] |
| O-1 | - | - | - | - | 71 | 2.05 | 16.2 | 6.3 | 14.3 | 16.6 | 1.47 | 0.24 | 0.94 | 0.46 | ns[3] |
| O-2 | -- | -- | -- | -- | 73 | 1.97 | 15.8 | 6.0 | 14.8 | 17.1 | 1.45 | 0.23 | 0.98 | 0.48 | ns[3] |
| P-1 | 234 | 39 | 6.0 | 2701 | 91 | 1.92 | 17.2 | 6.6 | 19.1 | 8.2 | 1.49 | 0.24 | 1.19 | 0.22 | ns[3] |
| P-2 | 143 | 25 | 5.8 | 2280 | 90 | 1.81 | 16.9 | 6.4 | 19.0 | 8.7 | 1.47 | 0.23 | 1.19 | 0.23 | ns[3] |

*viscosity measured as a 2.0 wt% solution of the esterified cellulose ether in 0.43 wt% aqueous NaOH at 20 °C
[1] Comparative Example A: HPMC of 6 mPa·s
[2] Comparative Example B: HPMC of 3 mPa·s
[3] Not entirely soluble

The results in Table 2 above illustrate that the hydroxyalkyl methyl cellulose acetate succinates of the present invention have a combination of a weight average molecular weight that makes them suitable as enteric polymers for pharmaceutical dosage forms and a low turbidity as 1.5 weight percent solution in acetone. Also, the measured weight average molecular weight $M_w$ of the produced HPMCAS was higher than could be expected based on the $M_w$ of the HPMC used as a starting material. The $M_w$ of the HPMC was about 20,000 Dalton. Taking the weight gain by the acetyl and succinoyl groups into account, an $M_w$ of about 25,000 Dalton (25 kDa) could be expected.

Comparative Example A is not directly comparable with Examples 1 - 10 because the HPMCAS of Comparative Example A has been produced from a HPMC of higher viscosity. Even a 1.5 wt.% solution of the HPMCAS of Comparative Example A in acetone was gel-like. Such HPMCAS would not be useful in end-uses such as spray-drying which typically start from solutions comprising at least 7 weight percent, more typically at least 10 weight percent of the hydroxyalkyl methyl cellulose acetate succinate.

The HPMCAS of Comparative Examples B - J have a low turbidity in acetone but an unfavorably low $M_w$.

The HPMCAS of Comparative Examples K - M have an $M_w$ within the range encompassed by Examples 1 - 10, but a considerably higher turbidity in acetone.

The HPMCAS of Comparative Examples N, O-1, O-2, P-1 and P-2 were not entirely soluble in acetone at a concentration of 1.5 wt-%. Small amounts of non-dissolved HPMCAS remained. Accordingly, the turbidity could not be reliably measured.

In the HPLC method utilized for determination of $M_w$, $M_n$ and $M_z$, in all Examples 1 - 10 and in Comparative Examples A to N a very good recovery rate ( = [weight of HPMCAS recovered from HPLC column / weight of HPMCAS introduced into HPLC column] x 100) was achieved, which allowed a reliable determination of $M_w$, $M_n$ and $M_z$. However, in Comparative Examples O-1 and O-2 the recovery rate was too low to make a reasonably reliable $M_w$, $M_n$ and $M_z$ determination.

**Claims**

1.    A hydroxyalkyl methyl cellulose acetate succinate

     a) having from 4.0 to less than 10.0 weight percent of succinoyl groups,
     b) having a weight average molecular weight $M_w$ of from 80,000 Dalton to 350,000 Dalton, and a z-average molecular weight $M_z$ of from 300,000 to 2,000,000 Dalton, $M_w$ and $M_z$ beingmeasured by SEC-MALLS using as mobile phase a mixture of 40 parts by volume of acetonitrile and 60 parts by volume of aqueous buffer containing 50 mM $NaH_2PO_4$ and 0.1 M $NaNO_3$,
     c) exhibiting a turbidity of up to 41 NTU as a 1.5 weight percent solution in acetone, measured according to USEPA method 180.1, and
     d) having a viscosity of up to 4.0 mPa·s, measured as a 2.0 wt% solution of the esterified cellulose ether in 0.43 wt.-% aqueous NaOH at 20 °C according to an Ubbelohde measurement according to DIN 51562-1:1999-01.

2.    The hydroxyalkyl methyl cellulose acetate succinate of claim 1 exhibiting a turbidity of up to 37 NTU as a 1.5 weight percent solution in acetone.

3.    The hydroxyalkyl methyl cellulose acetate succinate of claim 1 or claim 2 having e) from 10.0 to 14.0 weight percent of acetyl groups.

4.    A hydroxyalkyl methyl cellulose acetate succinate

     a) having from 10.0 to 20.0 weight percent of succinoyl groups,
     b) having a weight average molecular weight $M_w$ of from 80,000 Dalton to 350,000 Dalton, and a z-average molecular weight $M_z$ of from 300,000 to 2,000,000 Dalton, $M_w$ and $M_z$ being measured by SEC-MALLS using as mobile phase a mixture of 40 parts by volume of acetonitrile and 60 parts by volume of aqueous buffer containing 50 mM $NaH_2PO_4$ and 0.1 M $NaNO_3$,
     c) exhibiting a turbidity of up to 37 NTU as a 1.5 weight percent solution in acetone, measured according to USEPA method 180.1, and
     d) having a viscosity of up to 4.0 mPa·s, measured as a 2.0 wt% solution of the esterified cellulose ether in 0.43 wt.-% aqueous NaOH at 20 °C according to an Ubbelohde measurement according to DIN 51562-1:1999-01.

5.    The hydroxyalkyl methyl cellulose acetate succinate of claim 4 having e) from 5.0 to 11.0 weight percent of acetyl groups.

6. The hydroxyalkyl methyl cellulose acetate succinate of any one of claims 1 to 5 exhibiting a turbidity of from 10 to 30 NTU.

7. A composition comprising a liquid diluent and at least one hydroxyalkyl methyl cellulose acetate succinate of any one of claims 1 to 6.

8. The composition of claim 7 additionally comprising at least one active ingredient and optionally one or more adjuvants.

9. A solid dispersion comprising at least one active ingredient in at least one hydroxyalkyl methyl cellulose acetate succinate of any one of claims 1 to 6.

10. The solid dispersion of claim 9 in the form of strands, pellets, granules, pills, tablets, caplets, microparticles, fillings of capsules or injection molded capsules or in the form of a powder, film, paste, cream, suspension or slurry.

11. A process for producing the solid dispersion of claim 9 or 10 comprising the steps of blending a) at least one hydroxyalkyl methyl cellulose acetate succinate of any one of claims 1 to 6, b) one or more active ingredients and c) one or more optional additives, and subjecting the blend to extrusion.

12. A process for producing the solid dispersion of claim 9 or 10 comprising the steps of blending a) at least one hydroxyalkyl methyl cellulose acetate succinate of any one of claims 1 to 6, b) one or more active ingredients, c) one or more optional additives, and d) a liquid diluent to prepare a liquid composition, and removing said liquid diluent.

13. The process of claim 12 wherein the liquid composition is subjected to spray-drying.

14. A dosage form being coated with the hydroxyalkyl methyl cellulose acetate succinate of any one of claims 1 to 6.

15. A capsule shell comprising the hydroxyalkyl methyl cellulose acetate succinate of any one of claims 1 to 6.

**Patentansprüche**

1. Hydroxyalkylmethylcelluloseacetatsuccinat

   a) mit 4,0 bis weniger als 10,0 Gew.-% Succinoylgruppen,
   b) mit einem gewichtsmittleren Molekulargewicht $M_w$ von 80.000 Dalton bis 350.000 Dalton und einem z-mittleren Molekulargewicht $M_z$ von 300.000 bis 2.000.000 Dalton, wobei $M_w$ und $M_z$ durch SEC-MALLS unter Verwendung einer Mischung aus 40 Volumenteilen Acetonitril und 60 Volumenteilen eines wässrigen Puffers, der 50 mM $NaH_2PO_4$ und 0,1 M $NaNO_3$ enthält, als mobile Phase gemessen wird,
   c) das eine Trübe von bis zu 41 NTU in einer 1,5 gew.-%igen Lösung in Aceton, gemessen gemäß USEPA-Verfahren 180.1, zeigt, und
   d) mit einer Viskosität von bis zu 4,0 mPa·s, gemessen als 2,0 gew.-%ige Lösung des veresterten Celluloseethers in 0,43 gew.-%iger wässriger NAOH bei 20°C gemäß einer Ubbelohde-Messung nach DIN 51562-1:1999-01.

2. Hydroxylalkylmethylcelluloseacetatsuccinat nach Anspruch 1, das eine Trübe von bis zu 37 NTU, gemessen als 1,5 gew.-%ige Lösung in Aceton, zeigt.

3. Hydroxylalkylmethylcelluloseacetatsuccinat nach Anspruch 1 oder 2 mit e) von 10,0 bis 14,0 Gew.-% an Acetylgruppen.

4. Hydroxylalkylmethylcelluloseacetatsuccinat

   a) mit 10,0 bis 20,0 Gew.-% Succinoylgruppen,
   b) mit einem gewichtsmittleren Molekulargewicht $M_w$ von 80.000 Dalton bis 350.000 Dalton und einem z-mittleren Molekulargewicht $M_z$ von 300.000 bis 2.000.000 Dalton, wobei $M_w$ und $M_z$ durch SEC-MALLS unter Verwendung einer Mischung von 40 Volumenteilen Acetonitril und 60 Volumenteilen eines wässrigen Puffers, der 50 mM $NaH_2PO_4$ und 0,1 M $NaNO_3$ enthält, als mobile Phase gemessen wird,
   c) das eine Trübe von bis zu 37 NTU in einer 1,5 gew.-%igen Lösung in Aceton, gemessen gemäß USEPA-Verfahren 180.1 zeigt, und

d) mit einer Viskosität von bis zu 4,0 mPa·s, gemessen als 2,0 gew.-%ige Lösung des veresterten Celluloseethers in 0,43 gew.-%iger wässriger NAOH bei 20°C gemäß einer Ubbelohde-Messung nach DIN 51562-1:1999-01.

5. Hydroxylalkylmethylcelluloseacetatsuccinat nach Anspruch 4 mit e) 5,0 bis 11,0 Gew.-% an Acetylgruppen.

6. Hydroxylalkylmethylcelluloseacetatsuccinat nach einem der Ansprüche 1 bis 5, das eine Trübe von 10 bis 30 NTU zeigt.

7. Zusammensetzung enthaltend ein flüssiges Verdünnungsmittel und wenigstens ein Hydroxylalkylmethylcelluloseacetatsuccinat nach einem der Ansprüche 1 bis 6.

8. Zusammensetzung nach Anspruch 7, die zusätzlich wenigstens einen aktiven Bestandteil und optional einen oder mehrere Hilfsstoffe enthält.

9. Feste Dispersion, enthaltend wenigstens einen aktiven Bestandteil in wenigstens einem Hydroxylalkylmethylcelluloseacetatsuccinat nach einem der Ansprüche 1 bis 6.

10. Feste Dispersion nach Anspruch 9 in Form von Strängen, Pellets, Granalien, Pillen, Tabletten, Filmtabletten, Mikroteilchen, Füllungen von Kapseln oder spritzgegossenen Kapseln oder in Form eines Pulvers, Films, einer Paste, Creme Suspension oder Aufschlämmung.

11. Verfahren zur Herstellung der festen Dispersion nach Anspruch 9 oder 10, umfassend die Schritte von Mischen

   a) wenigstens eines Hydroxylalkylmethylcelluloseacetatsuccinats nach einem der Ansprüche 1 bis 6,
   b) eines oder mehrerer aktiven Bestandteile und
   c) eines oder mehrerer optionaler Additive und
   Unterwerfen der Mischung einer Extrusion.

12. Verfahren zur Herstellung der festen Dispersion nach Anspruch 9 oder 10, umfassend die Schritte von Mischen

   a) wenigstens eines Hydroxylalkylmethylcelluloseacetatsuccinats nach einem der Ansprüche 1 bis 6,
   b) eines oder mehrerer aktiven Bestandteile,
   c) eines oder mehrerer optionaler Additive und
   d) eines flüssigen Verdünnungsmittels, um eine flüssige Zusammensetzung herzustellen

und Entfernen dieses flüssigen Verdünnungsmittels.

13. Verfahren nach Anspruch 12, wobei die flüssige Zusammensetzung Sprühtrocknen unterzogen wird.

14. Dosierform, die mit dem Hydroxylalkylmethylcelluloseacetatsuccinat nach einem der Ansprüche 1 bis 6 beschichtet ist.

15. Kapselschale enthaltend das Hydroxylalkylmethylcelluloseacetatsuccinat nach einem der Ansprüche 1 bis 6.

**Revendications**

1. Un acétate succinate d'hydroxyalkylméthylcellulose

   a) ayant de 4,0 à moins de 10,0 pour cent en poids de groupes succinoyle,
   b) ayant une masse moléculaire moyenne en poids $M_w$ allant de 80 000 Daltons à 350 000 Daltons, et une masse moléculaire moyenne en z $M_z$ allant de 300 000 à 2 000 000 Daltons, $M_w$ et $M_z$ étant mesurées par SEC-MALLS en utilisant comme phase mobile un mélange de 40 parties en volume d'acétonitrile et de 60 parties en volume de tampon aqueux contenant 50 mM de $NaH_2PO_4$ et 0,1 M de $NaNO_3$,
   c) présentant une turbidité allant jusqu'à 41 UTN en solution à 1,5 pour cent en poids dans de l'acétone, mesurée selon la méthode USEPA 180.1, et
   d) ayant une viscosité allant jusqu'à 4,0 mPa·s, mesurée en solution à 2,0 % en poids de l'éther de cellulose estérifié dans 0,43 % en poids de NaOH aqueux à 20 °C selon une mesure Ubbelohde selon la DIN 51562-1:1999-01.

**2.** L'acétate succinate d'hydroxyalkylméthylcellulose de la revendication 1 présentant une turbidité allant jusqu'à 37 UTN en solution à 1,5 pour cent en poids dans de l'acétone.

**3.** L'acétate succinate d'hydroxyalkylméthylcellulose de la revendication 1 ou de la revendication 2 ayant e) de 10,0 à 14,0 pour cent en poids de groupes acétyle.

**4.** Un acétate succinate d'hydroxyalkylméthylcellulose

a) ayant de 10,0 à 20,0 pour cent en poids de groupes succinoyle,
b) ayant une masse moléculaire moyenne en poids $M_w$ allant de 80 000 Daltons à 350 000 Daltons, et une masse moléculaire moyenne en z $M_z$ allant de 300 000 à 2 000 000 Daltons, $M_w$ et $M_z$ étant mesurées par SEC-MALLS en utilisant comme phase mobile un mélange de 40 parties en volume d'acétonitrile et de 60 parties en volume de tampon aqueux contenant 50 mM de $NaH_2PO_4$ et 0,1 M de $NaNO_3$,
c) présentant une turbidité allant jusqu'à 37 UTN en solution à 1,5 pour cent en poids dans de l'acétone, mesurée selon la méthode USEPA 180.1, et
d) ayant une viscosité allant jusqu'à 4,0 mPa·s, mesurée en solution à 2,0 % en poids de l'éther de cellulose estérifié dans 0,43 % en poids de NaOH aqueux à 20 °C selon une mesure Ubbelohde selon la DIN 51562-1:1999-01.

**5.** L'acétate succinate d'hydroxyalkylméthylcellulose de la revendication 4 ayant e) de 5,0 à 11,0 pour cent en poids de groupes acétyle.

**6.** L'acétate succinate d'hydroxyalkylméthylcellulose de l'une quelconque des revendications 1 à 5 présentant une turbidité allant de 10 à 30 UTN.

**7.** Une composition comprenant un diluant liquide et au moins un acétate succinate d'hydroxyalkylméthylcellulose de l'une quelconque des revendications 1 à 6.

**8.** La composition de la revendication 7 comprenant en sus au moins un principe actif et facultativement un ou plusieurs adjuvants.

**9.** Une dispersion solide comprenant au moins un principe actif dans au moins un acétate succinate d'hydroxyalkylméthylcellulose de l'une quelconque des revendications 1 à 6.

**10.** La dispersion solide de la revendication 9 sous la forme de brins, de pastilles, de granules, de pilules, de comprimés, de comprimés-capsules, de microparticules, de matières de remplissage de capsules ou de capsules moulées par injection ou sous la forme d'une poudre, d'un film, d'une pâte, d'une crème, d'une suspension ou d'une suspension épaisse.

**11.** Un procédé pour produire la dispersion solide de la revendication 9 ou de la revendication 10 comprenant les étapes consistant à mélanger de façon homogène a) au moins un acétate succinate d'hydroxyalkylméthylcellulose de l'une quelconque des revendications 1 à 6, b) un ou plusieurs principes actifs et c) un ou plusieurs additifs facultatifs, et à soumettre le mélange homogène à une extrusion.

**12.** Un procédé pour produire la dispersion solide de la revendication 9 ou de la revendication 10 comprenant les étapes consistant à mélanger de façon homogène a) au moins un acétate succinate d'hydroxyalkylméthylcellulose de l'une quelconque des revendications 1 à 6, b) un ou plusieurs principes actifs, c) un ou plusieurs additifs facultatifs, et d) un diluant liquide afin de préparer une composition liquide, et à retirer ledit diluant liquide.

**13.** Le procédé de la revendication 12 dans lequel la composition liquide est soumise à un séchage par atomisation.

**14.** Une forme galénique qui est enrobée de l'acétate succinate d'hydroxyalkylméthylcellulose de l'une quelconque des revendications 1 à 6.

**15.** Une enveloppe de capsule comprenant l'acétate succinate d'hydroxyalkylméthylcellulose de l'une quelconque des revendications 1 à 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4365060 A
- US 4226981 A
- EP 0219426 A
- WO 2005115330 A
- WO 2011159626 A
- EP 1141029 A
- EP 210917 A
- EP 1423433 A
- US 4316982 A
- EP 0872233 A
- EP 0219426 A2
- US 5776501 A

**Non-patent literature cited in the description**

- Chemistry and applications of cellulosic polymers for enteric coatings of solid dosage forms. **WU S.H. W. ; WYATT D.M. ; ADAMS M. W.** Aqueous Coatings for Pharmaceutical Dosage Forms. Marcel Dekker, 1997, 385-418
- **EDGAR et al.** Cellulose esters in drug delivery. *Cellulose,* 2007, vol. 14, 49-64
- Hypromellose Acetate Succinate. *United States Pharmacopia and National Formulary,* vol. 29, 1548-1550
- Hypromellose. *United States Pharmacopeia and National Formulary,* vol. 35, 3467-3469
- *Journal of Pharmaceutical and Biomedical Analysis,* 2011, vol. 56, 743
- Hypromellose Acetate Succinate. *United States Pharmacopia and National Formulary, NF,* vol. 29, 1548-1550
- Perry's Chemical Engineers' Handbook. 1984, 20-54, 20-57
- **MARSHALL.** Atomization and Spray-Drying. *Chem. Eng. Prog. Monogr. Series,* 1954, vol. 50, 2
- **MASTERS.** Spray Drying Handbook. 1985
- **JOERG BREITENBACH.** Melt extrusion: from process to drug delivery technology. *European Journal of Pharmaceutics and Biopharmaceutics,* 2002, vol. 54, 107-117
- Hypromellose. *United States Pharmacopeia and National Formulary, USP,* vol. 35, 3467-3469
- **CHEN, R. et al.** *Journal of Pharmaceutical and Biomedical Analysis,* 2011, vol. 56, 743-748